# FASCICULE DE BREVET EUROPEEN

(11) **EP 1 277 462 B1**
(45) Date de publication et mention de la délivrance du brevet: **14.09.2005**
(21) Numéro de dépôt: 02291755.3
(22) Date de dépôt: 11.07.2002
(51) Int. Cl.: A61K 7/48

(54) **Composition autobronzante contenant un ester N-acyl- d'acide amine**
Selbstbräunendes Mittel enthaltend einen Ester von einer N-Acyl Aminosäure
Self- tanning composition containing an ester of an N-acyl amino acid

(30) Priorité: 19.07.2001 FR 0109673
(43) Date de publication de la demande: 22.01.2003
(73) Titulaire: L'OREAL, 75008 Paris (FR)
(72) Inventeur: Candau, Didier, 91570 Bi-vres (FR)
(74) Mandataire: Casalonga, Axel

(56) Documents cités:
- EP-A- 0 538 764
- EP-A- 0 913 390
- EP-A- 0 968 704
- EP-A- 1 044 676
- FR-A- 2 775 595

## Description

La présente invention se rapporte à une composition cosmétique et/ou dermatologique plus particulièrement destinée au bronzage et/ou au brunissage artificiels de la peau et comprenant, dans un support cosmétiquement acceptable, au moins un ester N-acylé d'acide aminé et au moins un agent autobronzant.

La présente invention se rapporte encore à un procédé de traitement cosmétique pour bronzer ou brunir artificiellement la peau et à l'utilisation d'un ester N-acylé d'acide aminé pour améliorer la coloration et/ou la stabilité d'un agent autobronzant.

L'invention concerne également les applications de ces compositions à la coloration de la peau proche du bronzage naturel de la peau.

"Par agent autobronzant" au sens de la présente demande, on entend un agent qui, appliqué sur la peau, notamment sur le visage, permet d'obtenir un effet de bronzage d'apparence plus ou moins semblable à celui qui peut résulter d'une exposition prolongée au soleil (bronzage naturel) ou sous une lampe UV.

De nos jours, il est important d'avoir bonne mine et une peau bronzée est toujours signe de bonne santé. Cependant, le bronzage naturel n'est pas toujours souhaitable dans la mesure où il nécessite des expositions prolongées aux rayonnements UV, en particulier aux rayonnements UV-A qui provoquent le brunissement de la peau mais en contrepartie sont susceptibles d'induire des réactions voire une altération de celle-ci, notamment dans le cas d'une peau sensible ou d'une peau continuellement exposée au rayonnement solaire : érythème, brûlure, perte d'élasticité, apparition de rides, vieillissement prématuré. Il est donc souhaitable de trouver une alternative au bronzage naturel qui soit compatible avec les exigences de telles peaux.

La plupart des produits cosmétiques destinés au bronzage artificiel de la peau sont à base de dérivés carbonylés permettant, par interaction avec les acides aminés de la peau, la formation de produits colorés, parmi ceux-ci on compte des composés mono- ou polycarbonylés tels que par exemple l'isatine, l'alloxane, la ninhydrine, le glycéraldéhyde, l'aldéhyde mésotartrique, la glutaraldéhyde, l'érythrulose, la dihydroxyacétone (DHA).

La DHA, est un produit particulièrement intéressant qui est couramment utilisé en cosmétique comme agent de bronzage artificiel de la peau ; appliqué sur cette dernière, notamment sur le visage, il permet d'obtenir un effet de bronzage ou de brunissage d'apparence semblable à celui qui peut résulter d'une exposition prolongée au soleil (bronzage naturel) ou sous une lampe UV.

Un inconvénient de la DHA est la lenteur avec laquelle la coloration se développe : il faut en effet compter plusieurs heures (3 à 5 heures en général) pour que soit révélée la coloration. L'intensité de la coloration obtenue sur la peau et /ou sa tenue au cours du temps (résistance au lavage) et/ou la rapidité avec laquelle la coloration se développe, sont souvent jugées insuffisantes par les utilisateurs de compositions autobronzantes à base de DHA.

Un autre problème posé par les compositions à base de DHA est qu'elles présentent la fâcheuse tendance, plus ou moins prononcée selon la nature du milieu dans lequel elles sont formulées, à se dégrader au cours du temps. Ces problèmes liés au stockage et/ou à la conservation des compositions à base de DHA se traduisent généralement à terme par un jaunissement non souhaitable de ces compositions.

Il existe donc une demande croissante de produits autobronzants agissant rapidement et conférant une coloration proche du bronzage naturel.

De manière surprenante et avantageuse, la demanderesse a trouvé que l'utilisation d'un ester N-acylé d'acide aminé permettait d'améliorer la stabilité et la coloration des compositions comprenant un agent autobronzant. Les colorations obtenues sont plus chromatiques, plus stables dans le temps, elles ont également une bonne résistance à l'eau et une bonne homogénéité.

Les compositions selon la présente invention présentent encore l'avantage de posséder des propriétés cosmétiques améliorées : elles confèrent à la peau une sensation de douceur et de fraîcheur, et permettent d'éviter un dessèchement de la peau ainsi qu'un toucher trop gras.

La composition selon la présente invention comprend dans un support cosmétiquement acceptable au moins un ester N-acylé d'acide aminé et au moins un agent autobronzant.

La présente demande a encore pour objet l'utilisation de la composition selon l'invention à titre de composition destinée au bronzage ou au brunissement de la peau ; et un procédé cosmétique de bronzage ou brunissement de la peau tel qu'il consiste à appliquer sur la peau une quantité efficace d'une composition selon l'invention.

Enfin, la présente demande concerne encore l'utilisation d'au moins un ester N-acylé d'acide aminé dans des compositions pour le bronzage et/ou le brunissage artificiels de la peau contenant au moins un agent autobronzant en vue d'améliorer la coloration et/ou la stabilité dudit agent autobronzant.

Les compositions conformes à l'invention permettent d'obtenir une coloration artificielle proche du bronzage naturel en un laps de temps court. Ainsi, on obtient une coloration immédiate qui permet une visualisation de l'application et par conséquent une meilleure homogénéité dans l'étalement de la composition sur la peau et donc de la coloration qui en résulte. De plus, la coloration artificielle obtenue sur la peau selon l'invention est extrêmement proche du bronzage naturel.

D'autres caractéristiques, aspects et avantages de la présente invention apparaîtront à la lecture de la description détaillée qui va suivre.

L'ester N-acylé d'acide aminé utilisé dans la présente invention est un ester de formule :

R'₁(CO)N(R'₂)CH(R'₃)(CH₂)ₙ(CO)OR'₄

dans laquelle :
n est un entier égal à 0, 1 ou 2,
R'₁ représente un radical en C₅ à C₂₁, linéaire ou ramifié, alkyle ou alcényle ,
R'₂ représente un atome d'hydrogène ou un groupe alkyle en C₁ à C₃,
R'₃ représente un radical choisi dans le groupe formé par un atome d'hydrogène, un groupe méthyle, un groupe éthyle, un radical alkyle linéaire ou ramifié en C₃ ou C₄,
R'₄ représente un radical alkyle en C₁ à C₁₀ linéaire ou ramifié, ou un radical alcényle en C₂ à C₁₀ linéaire ou ramifié ou un reste stérol.

Dans la formule ci-dessus, le groupement R'₁(CO)- est un groupement acyle d'un acide choisi de préférence dans le groupe formé par l'acide caprique, l'acide laurique, l'acide myristique, l'acide palmitique, l'acide stéarique, l'acide béhénique, l'acide linoléique, l'acide linolénique, l'acide oléique, l'acide isostéarique, l'acide 2-éthylhexanoïque, les acides gras d'huile de coco, les acides gras d'huile de palmiste, les acides gras hydrogénés d'huile de palmiste. Ces acides gras peuvent en outre présenter un groupe hydroxyle. De manière encore plus préférée, il s'agira de l'acide laurique.

La partie -N(R'₂)CH(R'₃)(CH₂)ₙ(CO)- de l'ester N-acylé d'acide aminé est de préférence choisie parmi les aminoacides suivants : glycine, alanine, valine, leucine, isoleucine, sérine, thréonine, proline, hydroxyproline, β-alanine, acide aminobutyrique, acide aminocaproïque, sarcosine, ou N-méthyl-β-alanine.

De manière encore plus préférée, il s'agira de la sarcosine.

La partie des esters N-acylés d'acide aminé correspondant au groupe OR'₄ peut être obtenue à partir des alcools choisis dans le groupe formé par le méthanol, éthanol, propanol, isopropanol, butanol, tert-butanol, isobutanol, 3-méthyl-1-butanol, 2-méthyl-1-butanol, huile de fusel, pentanol, héxanol, cyclohéxanol, octanol, 2-éthylhéxanol, décanol, alcool laurylique, alcool myristique, alcool cétylique, alcool cétostéaryl, alcool stéaryl, alcool oléique, alcool béhénylique, alcool de jojoba, alcool 2-héxadécylique, alcool 2-octyldodécanol et alcool isostéarylique.

Ces esters N-acylés d'acide aminé peuvent en particulier être obtenus à partir de sources naturelles en acides aminés. Dans ce cas, les acides aminés proviennent d'hydrolyse de protéines naturelles végétales (avoine, blé, soja, palme, coco) et conduisent alors nécessairement à des mélanges d'acides aminés qui devront ensuite être estérifiés puis N-acylés. La préparation de tels acides aminés est plus particulièrement décrite dans la demande de brevet FR 2 796 550.

L'ester d'acide aminé plus particulièrement préféré pour son utilisation dans la présente invention est le N-lauroylsarcosinate d'isopropyle de formule :

CH₃-(CH₂)₁₀CO-N(CH₃)-CH₂-COO- CH₂-(CH₃)₂.

Ces esters d'aminoacide, ainsi que leur procédé de synthèse sont décrits dans les demandes de brevet EP 1 044 676 et EP 0 928 608 de la société Ajinomoto Co.

Les agents autobronzants sont généralement choisis parmi les composés mono ou polycarbonylés tels que par exemple l'isatine, l'alloxane, la ninhydrine, le glycéraldéhyde, l'aldéhyde mésotartrique, la glutaraldéhyde, l'érythrulose, les dérivés de pyrazolin-4,5-diones telles que décrites dans la demande de brevet FR 2 466 492 et WO 97/35842, la dihydroxyacétone (DHA), les dérivés de 4,4-dihydroxypyrazolin-5-ones telles que décrites dans la demande de brevet EP 903 342.

Les esters N-acylés d'acide aminé sont de préférence présents, dans les compositions de l'invention dans des concentrations allant de 0,5 à 50% en poids et plus particulièrement de 5 à 30 % en poids par rapport au poids total de la composition.

Dans un mode de réalisation particulièrement préféré de l'invention, on utilisera plus particulièrement la dihydroxyacétone (DHA).

La DHA peut être utilisée sous forme libre et/ou encapsulée par exemple dans des vésicules lipidiques telle que des liposomes, notamment décrits dans la demande WO 97/25970.

Ces agents autobronzants peuvent être associés à au moins un colorant direct synthétique ou naturel et/ou au moins un dérivé indolique comme ceux décrits dans les brevets EP 425 324 et EP 456 545.

Ces agents autobronzants peuvent également être associés à d'autres agents de coloration de la peau synthétiques ou naturels.

Au sens de la présente invention, on entendra par « agent de coloration de la peau », tout composé ayant une affinité particulière pour la peau lui permettant de conférer à cette dernière une coloration durable, non-couvrante (à savoir n'ayant pas tendance à opacifier la peau) et qui ne s'élimine ni à l'eau ni à l'aide d'un solvant, et qui résiste à la fois au frottement et au lavage par une solution contenant des tensioactifs. Une telle coloration durable se distingue donc de la coloration superficielle et momentanée apportée par exemple par un pigment de maquillage.

Les agents de coloration additionnels peuvent également être choisis par exemple parmi les extraits végétaux comme par exemple les extraits de bois rouges « insolubles » du genre Pterocarpus et du genre Baphia comme le Pterocarpus santalinus, le Pterocarpus osun, le Pterocarpus soyauxii, le Pterocarpus erinaceus, le Pterocarpus indicus ou encore le Baphia nitida comme ceux décrits dans la demande de brevet EP 971 683.

Les agents de coloration peuvent également être des nanopigments d'oxyde de fer dont la taille moyenne des particules élémentaires est inférieure à 100nm tels que ceux décrits dans la demande de brevet EP 966 953.

Les agents autobronzants sont généralement présents dans les compositions selon l'invention dans des proportions allant de 0,1 à 10% en poids par rapport au poids total de la composition, et de préférence de 0,2 à 8% en poids par rapport au poids total de la composition.

Les compositions autobronzantes conformes à l'invention peuvent se présenter sous forme de crèmes, de laits, de gels, de gel-crèmes, d'émulsions huile-dans-eau, de dispersions vésiculaires, de lotions fluides, en particulier de lotions fluides vaporisables ou tout autre forme généralement utilisée en cosmétique, en particulier celles convenant habituellement aux compositions cosmétiques autobronzantes.

Les compositions conformes à la présente invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les antagonistes de substance P, les anti-inflammatoires, les parfums, les conservateurs, les tensioactifs, les charges, les polymères autres que ceux de l'invention, les propulseurs, les agents alcanisants ou acidifiants, les colorants ou tout autre ingrédient habituellement utilisé dans le domaine cosmétique et/ou dermatologique, en particulier pour la fabrication de compositions autobronzantes sous forme d'émulsions.

Les corps gras peuvent être constitués par une huile ou une cire ou leurs mélanges. Par « huile », on entend un composé liquide à température ambiante. Par « cire », on entend un composé solide ou substantiellement solide à température ambiante, et dont le point de fusion est généralement supérieur à 35°C.

Comme huiles, on peut citer les huiles minérales (paraffine) ; végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société Finetex, le palmitate d'octyle, le lanolate d'isopropyle, les triglycérides dont ceux des acides caprique/caprylique, les esters et les éthers gras oxyéthylénés ou oxypropylénés ; siliconées (cyclométhicone, polydiméthyl-siloxanes ou PDMS) ou fluorées ; les polyalkylènes et leurs mélanges.

Comme composés cireux, on peut citer la paraffine, la cire de carnauba, la cire d'abeille, l'huile de ricin hydrogénée.

Parmi les solvants organiques, on peut citer les alcools et polyols inférieurs ayant au plus 8 atomes de carbone.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthylcellulose.

Les compositions conformes à l'invention peuvent comporter en plus au moins un agent photoprotecteur organique et/ou au moins un agent photoprotecteur inorganique actifs dans l'UVA et/ou l'UVB (absorbeurs), hydrosolubles ou liposolubles ou bien insolubles dans les solvants cosmétiques couramment utilisés.

Les agents photoprotecteurs organiques sont notamment choisis parmi les anthranilates ; les dérivés cinnamiques ; les dérivés de dibenzoylméthane ; les dérivés salicyliques ;les dérivés du camphre ; les dérivés de triazine tels que ceux décrits dans les demandes de brevet US 4,367,390, US 4,724,137, EP 863 145, EP 517 104, EP 570 838, EP 796 851, EP 775 698, EP 878 469, EP 933 376, EP 507 691, EP 507 692 , EP 790 243 et EP 944 624 ; les dérivés de la benzophénone ; les dérivés de β-β'-diphénylacrylate; les dérivés de benzotriazole ; les dérivés de benzalmalonate ; les dérivés de benzimidazole ; les imadazolines ; les dérivés bis-benzoazolyle tels que décrits dans les brevets EP 669 323 et US 2,463,264 ; les dérivés de l'acide p-aminobenzoïque (PABA) ; les dérivés de méthylène bis-(hydroxyphényl benzotriazole) tels que décrits dans les demandes US 5,237,071, US 5,166,355, GB 2303546, DE 19726184 et EP 893 119 ;les polymères filtres et silicones filtres tels que ceux décrits notamment dans la demande WO 93/04665 ; les dimères dérivés d'α-alkylstyrène tels que ceux décrits dans la demande de brevet DE 19855649 ; les 4,4-diarylbutadiènes tels que décrits dans les demandes EP 0 967 200 et DE 19755649 et leurs mélanges.

Comme exemples d'agents photoprotecteurs organiques actifs dans l'UV-A et/ou l'UV-B, on peut citer désignés ci-dessus sous leur nom INCI :

### Dérivés de l'acide para-aminobenzoïque :

- PABA,
- Ethyl PABA,
- Ethyl Dihydroxypropyl PABA,
- Ethylhexyl Diméthyl PABA vendu notamment sous le nom « ESCALOL 507 » par ISP,
- Glyceryl PABA,
- PEG-25 PABA vendu sous le nom « UVINUL P25 » par BASF.

### Dérivés salicyliques :

- Homosalate vendu sous le nom « EUSOLEX HMS » par RONA/EM INDUSTRIES,
- Ethylhexyl Salicylate vendu sous le nom « NEO HELIOPAN OS » par HAARMANN et REIMER,
- Dipropyleneglycol Salicylate vendu sous le nom « DIPSAL » par SCHER,
- TEA Salicylate vendu sous le nom « NEO HELIOPAN TS » par HAARMANN et REIMER.

### Dérivés du dibenzoylméthane :

- Butyl Methoxydibenzoylmethane vendu notamment sous le nom commercial « PARSOL 1789 » par HOFFMANN LAROCHE,
- Isopropyl Dibenzoylmethane.

### Dérivés cinnamiques :

- Ethylhexyl Methoxycinnamate vendu notamment sous le nom commercial « PARSOL MCX » par HOFFMANN LAROCHE,
- Isopropyl Methoxy cinnamate,
- Isoamyl Methoxy cinnamate vendu sous le nom commercial « NEO HELIOPAN E 1000 » par HAARMANN et REIMER,
- Cinoxate,
- DEA Methoxycinnamate,
- Glyceryl Ethylhexanoate Dimethoxycinnamate.

### Dérivés de β-β'-diphénylacrylate :

- Octocrylene vendu notamment sous le nom commercial « UVINUL N539 » par BASF,
- Etocrylene vendu notamment sous le nom commercial « UVINUL N35 » par BASF.

### Dérivés de la benzophénone :

- Benzophenone-1 vendu sous le nom commercial « UVINUL 400 » par BASF,
- Benzophenone-2 vendu sous le nom commercial « UVINUL D50 » par BASF,
- Benzophenone-3 ou Oxybenzone vendu sous le nom commercial « UVINUL M40 » par BASF,
- Benzophenone-4 vendu sous le nom commercial « UVINUL MS40 » par BASF,
- Benzophenone-5,
- Benzophenone-6 vendu sous le nom commercial « HELISORB 11 » par NORQUAY,
- Benzophenone-8 vendu sous le nom commercial « SPECTRA-SORB UV-24 » par AMERICAN CYANAMID,
- Benzophenone-9 vendu sous le nom commercial « UNIVUL DS-49 » par BASF,
- Benzophenone-12.

### Dérivés du benzylidène camphre :

- 3-Benzylidene camphor fabriqué sous le nom « MEXORYL SD » par CHIMEX,
- 4-Methylbenzylidene camphor vendu sous le nom « EUSOLEX 6300 » par MERCK,
- Benzylidene Camphor Sulfonic Acid fabriqué sous le nom « MEXORYL SO » par CHIMEX,
- Camphor Benzalkonium Methosulfate fabriqué sous le nom « MEXORYL SO » par CHIMEX,
- Terephthalylidene Dicamphor Sulfonic Acid fabriqué sous le nom « MEXORYL SX »par CHIMEX,
- Polyacrylamidomethyl Benzylidene Camphor fabriqué sous le nom « MEXORYL SW » par CHIMEX.

### Dérivés de benzimidazole :

- Phenylbenzimidazole Sulfonic Acid vendu notamment sous le nom commercial « EUSOLEX 232 » par MERCK,
- Benzimidazilate vendu sous le nom commercial « NEO HELIOPAN AP » par HAARMANN et REIMER.

### Dérivés de triazine :

- Anisotriazine vendu sous le nom commercial « TINOSORB S » par CIBA SPECIALITY CHEMICALS,
- Ethylhexyl triazone vendu notamment sous le nom commercial « UVINUL T150 » par BASF,
- Diethylhexyl Butamido Triazone vendu sous le nom commercial « UVASORB HEB » par SIGMA 3V,
- La 2,4,6-tris-(4'-amino benzalmalonate de diisobutyle)-s-triazine.

### Dérivés de benzotriazole :

- Drometrizole Trisiloxane vendu sous le nom « SILATRIZOLE » par RHODIA CHIMIE,
- Methylène bis-Benzotriazol Tetramethylbutylphénol vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALITY CHEMICALS.

### Dérivés anthraniliques :

- Menthyl anthranilate vendu sous le nom commercial « NEO HELIOPAN MA » par HAARMANN et REIMER.

### Dérivés d'imidazolines :

- Ethylhexyl Dimethoxybenzylidene Dioxoimidazoline Propionate.

### Dérivés de benzalmalonate :

- Polyorganosiloxane à fonctions benzalmalonate vendu sous la dénomination commerciale « PARSOL SLX » par HOFFMANN LAROCHE
et leurs mélanges.

Les agents photoprotecteurs organiques plus particulièrement préférés sont choisis parmi les composés suivants :
- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalydene Dicamphor Sulfonic Acid,
- Benzophenone-3,
- Benzophenone-4,
- Benzophenone-5,
- 4-Methylbenzylidene Camphor,
- Benzimidazilate,
- Anisotriazine,
- Ethylhexyl triazone,
- Diethylhexyl Butamido Triazone,
- Methylene bis-Benzotriazolyl Tetramethylbutylphenol,
- Drometrizole Trisiloxane,
- La 2,4,6-tris-(4'-amino benzalmalonate de diisobutyle)-s-triazine
et leurs mélanges.

Les agents photoprotecteurs inorganiques sont choisis parmi les pigments ou bien encore des nanopigments (taille moyenne des particules primaires : généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium et leurs mélanges. Des agents d'enrobage classiques sont par ailleurs l'alumine et/ou le stéarate d'aluminium. de tels nanopigments d'oxydes métalliques, enrobés ou non enrobés, sont en particulier décrits dans les demandes de brevets EP 518 772 et EP 518 773.

Les agents photoprotecteurs sont généralement présents dans les compositions selon l'invention dans des proportions allant de 0,1 à 20% en poids par rapport au poids total de la composition, et de préférence allant de 0,2 à 15% en poids par rapport au poids total de la composition.

Bien entendu, l'homme de l'art veillera à choisir le ou les éventuels composés complémentaires cités ci-dessus et/ou leurs quantités de manière telle que les propriétés avantageuses attachées intrinsèquement à l'association conforme à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait ou sous la forme d'un gel ou d'un gel crème, sous la forme d'une lotion, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans-huile.

Lorsqu'il s'agit d'une émulsion, la phase aqueuse de celle-ci peut comprendre une dispersion vésiculaire non ionique préparée selon les procédés connus (Bangham, Standish and Watkins J. Mol. Biol. 13, 238 (1965), FR 2 315 991 et FR 2 416 008).

L'invention concerne également un procédé de traitement cosmétique pour bronzer et/ou brunir artificiellement la peau, caractérisé par le fait qu'il consiste à appliquer sur celle-ci une quantité efficace d'une composition cosmétique telle que définie ci-dessus.

L'invention concerne également l'utilisation d'un ester N-acylé d'acide aminé tel que défini précédemment dans le but d'améliorer la coloration et/ou la stabilité d'un agent autobronzant tels que ceux définis ci-dessus contenu dans une composition cosmétique destinée au bronzage et/ou brunissage artificiels de la peau.

Des exemples concrets, mais nullement limitatifs, illustrant l'invention, vont maintenant être donnés.

### EXEMPLES

### EXEMPLE 1

| **COMPOSITION EMULSION H/E** | **% EN POIDS** |
|---|---|
| Mélange mono/distéarate de glycerol / stéarate de poyéthylène glycol (100 OE) | 2 |
| (ARLACEL 165 FL- ICI) | |
| Alcool stéarylique | 1 |
| (LANETTE 18 - HENKEL) | |
| Acide stéarique d'huile de palme | 1,5 |
| (STEARINE TP - STEARINERIE DUBOIS) | |
| Poly diméthylsiloxane | 2 |
| (DOW CORNING 200 FLUID - DOW CORNING) | |
| Mélange poly diméthylsiloxane alpha-omega dihydroxyle/cyclopentadiméthylsiloxane (14,7/85,3) | 3 |
| (DOW CORNING 1501 FLUID - DOW CORNING) | |
| Lauroyl sarcosinate d'isopropyle | 15 |
| (ELDEW SL205-AJINOMOTO) | |
| Dihydroxyacétone | 5 |
| Glycérine | 5 |
| Phosphate d'alcool hexadécylique, sel de potassium | 1 |
| (AMPHISOL K - HOFFMANN LA ROCHE) | |
| Acide polyacrylique | 0,3 |
| (SYNTHALEN K - 3V) | |
| Triéthanolamine | Qs pH : 7 |
| Acétate de DL-ALPHA-TOCOPHERYLE | 0,5 |
| (HOFFMANN LA ROCHE) | |
| Conservateurs | Qs |
| Eau déminéralisée qsq | 100g |

### EXEMPLE 2

| **COMPOSITION EMULSION E/H** | **% EN POIDS** |
|---|---|
| Polydiméthyl/méthylcétyl méthylsiloxane oxyéthyléné | 2 |
| (ABIL EM 90D - GOLDSCHMIDT) | |
| Phényl triméthylsiloxy trisiloxane | 3 |
| (DOW CORNING 556 COSMETIC grade fluid - DOW CORNING) | |
| Lauroyl sarcosinate d'isopropyle | 10 |
| (ELDEW SL205-AJINOMOTO) | |
| ISOHEXADECANE | 5 |
| (PERMETHYL 101 A - BAYER - GENERAL BAYER SILICONES-SILICONES) | |
| CYCLOPENTA DIMETHYLSILOXANE | 3 |
| (DC 24'5 FLUID - DOW CORNING) | |
| Dihydroxyacétone | 3 |
| (MERCK) | |
| Glycérine | 5 |
| Sulfate de magnésium | 0,7 |
| Conservateurs | qs |
| Eau déminéralisée qsq | 100 g |

## Revendications

1. Composition cosmétique et/ou dermatologique, **caractérisée par le fait qu'**elle contient dans un support cosmétiquement acceptable :
(i) au moins un agent autobronzant,
(ii) au moins un ester choisi parmi les esters N-acylés d'acide aminé de formule
R'₁(CO)N(R'₂)CH(R'₃)(CH₂)ₙ(CO)OR'₄
dans laquelle :
n est un entier égal à 0,1 ou 2,
R'₁ représente un radical alkyle ou alcényle en C₅ à C₂₁, linéaire ou ramifié,
R'₂ représente un atome d'hydrogène ou un groupe alkyle en C₁ à C₃,
R'₃ représente un radical choisi dans le groupe formé par un atome d'hydrogène, un groupe méthyle, un groupe éthyle, une chaîne alkyle linéaire ou ramifiée en C₃ ou C₄,
R'₄ représente un radical alkyle en C₁ à C₁₀ linéaire ou ramifié, ou un radical alcényle en C₂ à C₁₀ linéaire ou ramifié ou un reste stérol.

2. Composition selon la revendication 1, **caractérisée en ce que** l'ester d'acide aminé est le N-lauroylsarcosinate d'isopropyle
CH₃-(CH₂)₁₀CO-N(CH₃)-CH₂-COO- CH₂-(CH₃)₂.

3. Composition selon l'une des revendications précédentes, telle que l'agent autobronzant est un composé mono ou polycarbonylé.

4. Composition selon la revendication 3, telle que l'agent autobronzant est choisi dans le groupe formé par l'isatine, l'alloxane, la ninhydrine, le glycéraldéhyde, l'aldéhyde mésotartrique, la glutaraldéhyde, l'érythrulose, les dérivés de pirazoline -4,5-dione, la dihydroxyacétone (DHA), les dérivés de 4,4-dihydroxypirazoline-5-diones.

5. Composition selon la revendication 4, telle que l'agent autobronzant est la DHA.

6. Composition selon l'une quelconque des revendications 1 à 5, telle que la concentration en agents autobronzants varie de 0,1 à 10% en poids, par rapport au poids total de la composition.

7. Composition selon l'une des revendications précédentes, **caractérisée par le fait qu'**elle comporte au moins un colorant direct, synthétique ou naturel, et/ou au moins un dérivé indolique.

8. Composition selon l'une des revendications précédentes, telle qu'elle comprend un agent de coloration additionnel choisi parmi les extraits de bois rouges "insolubles" du genre Pterocarpus et du genre Baphia .

9. Composition selon l'une des revendications précédentes, telle qu'elle comprend un agent de coloration additionnel choisi parmi les nanopigments d'oxyde de fer dont la taille moyenne des particules élémentaires est inférieure à 100 nm.

10. Composition selon l'une quelconque des revendications précédentes, **caractérisée par le fait qu'**elle comporte au moins un adjuvant cosmétique choisi parmi les corps gras, les solvants organiques, les émulsionnants, les épaississants ioniques ou non ioniques, les adoucissants, les antioxydants, les agents anti-radicaux libres, les opacifiants, les stabilisants, les émollients, les silicones, les α-hydroxyacides, les agents anti-mousse, les agents hydratants, les vitamines, les agents répulsifs contre les insectes, les antagonistes de substance P, les anti-inflammatoires, les parfums, les conservateurs, les tensidactifs, les charges, les polymères, les propulseurs, les agents alcalinisants ou acidifiants.

11. Composition selon l'une quelconque des revendications précédentes, **caractérisée par le fait qu'**elle comporte en plus en outre au moins un agent photoprotecteur organique et/ou au moins un agent photoprotecteur inorganique actifs dans l'UVA et/ou dans l'UVB.

12. Composition selon la revendication 11, telle que l'agent photoprotecteur organique est choisi parmi les dérivés 1,3,5-triazine, dérivés du dibenzoylméthane, les dérivés cinnamiques, les anthranilates ; les dérivés salicyliques, les dérivés du camphre ; les dérivés de la benzophénone ; les dérivés de β,β-diphénylacrylate, les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-hydroxyphényl benzotriazole ; les polymères filtres et silicones filtres; les dimères dérivés d'α-alkylstyrène et les 4,4-diarylbutadiènes et leurs mélanges.

13. Composition selon la revendication 12, telle que l'agent photoprotecteur organique est choisi parmi :
- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalydene Dicamphor Sulfonic Acid,
- Benzophenone-3,
- Benzophenone-4,
- Benzophenone-5,
- 4-Methylbenzylidene Camphor,
- Benzimidazilate,
- Anisotriazine,
- Ethylhexyl triazone,
- Diethylhexyl Butamido Triazone,
- Methylene bis-Benzotriazolyl Tetramethylbutylphenol,
- Drometrizole Trisiloxane,
- La 2,4,6-tris-(4'-amino benzalmalonate de diisobutyle)-s-triazine
et leurs mélanges.

14. Composition selon la revendication 11, telle que l'agent photoprotecteur inorganique est choisi parmi les pigments ou les nanopigments d'oxydes métalliques enrobés ou non.

15. Composition selon la revendication 14, telle que l'agent photoprotecteur inorganique est choisi parmi les nanopigments enrobés ou non, d'oxyde de titane, de fer, de zinc, de zirconium ou de cérium et leurs mélanges.

16. Composition selon l'une quelconque des revendications 11 à 15 telle que l'agent photoprotecteur est présent dans les compositions dans des proportions allant de 0,1 à 20% en poids par rapport au poids total de la composition, et de préférence allant de 0,2 à 15% en poids par rapport au poids total de la composition.

17. Composition selon l'une des revendications précédentes, **caractérisée en ce qu'**elle comprend de 0,5 à 50%, de préférence de 5 à 30% de dérivé d'ester N-acylés d'acide aminé en poids par rapport au poids total de la composition.

18. Composition selon l'une des revendications précédentes, telle qu'elle se présente sous la forme d'une dispersion vésiculaire non ionique, d'une émulsion, en particulier d'une émulsion de type eau-dans huile, de type huile-dans-eau, d'une crème, ou d'une émulsion triple (E/H/E ou H/E/H), d'un lait, d'un gel, d'un gel crème, d'une suspension, d'une dispersion, d'une mousse ou d'un spray.

19. Utilisation d'une composition selon l'une des revendications 1 à 18 à titre de composition cosmétique destinée au bronzage ou au brunissement de la peau.

20. Procédé cosmétique de bronzage ou brunissement de la peau **caractérisé en ce qu'**il consiste à appliquer sur la peau une quantité efficace d'une composition cosmétique selon l'une des revendications 1 à 18.

21. Utilisation d'au moins un ester N-acylé d'acide aminé de formule
R'₁(CO)N(R'₂)CH(R'₃)(CH₂)ₙ(CO)OR'₄
dans laquelle :
n est un entier égal à 0,1 ou 2,
R'₁ représente un radical alkyle ou alcényle en C₅ à C₂₁, linéaire ou ramifié,
R'₂ représente un atome d'hydrogène ou un groupe alkyle en C₁ à C₃,
R'₃ représente un radical choisi dans le groupe formé par un atome d'hydrogène, un groupe méthyle, un groupe éthyle, une chaîne alkyle linéaire ou ramifiée en C₃ ou C₄,
R'₄ représente un radical alkyle en C₁ à C₁₀ linéaire ou ramifié, ou un radical alcényle en C₂ à C₁₀ linéaire ou ramifié ou un reste stérol,
dans une composition pour le bronzage et/ou le brunissage artificiels de la peau contenant au moins un agent autobronzant dans le but d'améliorer la coloration et/ou la stabilité dudit agent autobronzant.

## Patentansprüche

1. Kosmetische und/oder dermatologische Zusammensetzung, **dadurch gekennzeichnet, dass** sie in einem kosmetisch akzeptablen Träger enthält:
(i) mindestens ein Selbstbräunungsmittel,
(ii) mindestens einen Ester, der unter den N-acylierten Aminosäureestern der folgenden Formel ausgewählt ist:
R'₁(CO)N(R'₂)CH(R'₃)(CH₂)ₙ(CO)OR'₄,
worin bedeuten:
n 0,1 oder 2,
R'₁ eine geradkettige oder verzweigte Alkyl- oder Alkenylgruppe mit 5 bis 21 Kohlenstoffatomen,
R'₂ ein Wasserstoffatom oder eine Alkylgruppe mit 1 bis 3 Kohlenstoffatomen,
R'₃ eine Gruppe, die unter Wasserstoff, Methyl, Ethyl oder einer geradkettigen oder verzweigten C₃- oder C₄-Alkylgruppe ausgewählt ist,
R'₄ eine geradkettige oder verzweigte Alkylgruppe mit 1 bis 10 Kohlenstoffatomen oder eine geradkettige oder verzweigte Alkenylgruppe mit 2 bis 10 Kohlenstoffatomen oder einen Sterinrest.

2. Zusammensetzung nach Anspruch 1, **dadurch gekennzeichnet, dass** der Aminosäureester das Isopropyl-N-lauroylsarcosinat
CH₃-(CH₂)₁₀CO-N(CH₃)-CH₂-COO-CH₂-(CH₃)₂
ist.

3. Zusammensetzung nach einem der vorhergehenden Ansprüche, **dadurch gekennzeichnet, dass** das Selbstbräunungsmittel eine Verbindung mit einer oder mehreren Carbonylgruppen ist.

4. Zusammensetzung nach Anspruch 3, **dadurch gekennzeichnet, dass** das Selbstbräunungsmittel unter Isatin, Alloxan, Ninhydrin, Glycerinaldehyd, meso-Weinsäurealdehyd, Glutaraldehyd, Erythrulose, Pirazolin-4,5-dionderivaten, Dihydroxyaceton (DHA) und 4,4-Dihydroxypirazolin-5-dionderivaten ausgewählt ist.

5. Zusammensetzung nach Anspruch 4, wobei das Selbstbräunungsmittel das DHA ist.

6. Zusammensetzung nach einem der Ansprüche 1 bis 5, wobei die Konzentration des Selbstbräunungsmittels im Bereich von 0,1 bis 10 Gew.-%, bezogen auf das Gesamtgewicht der Zusammensetzung, liegt.

7. Zusammensetzung nach einem der vorhergehenden Ansprüche, **dadurch gekennzeichnet, dass** sie mindestens einen synthetischen oder natürlichen Direktfarbstoff und/oder mindestens ein Indolderivat enthält.

8. Zusammensetzung nach einem der vorhergehenden Ansprüche, wobei sie ein weiteres Farbmittel enthält, das unter den "unlöslichen" Rotholzextrakten der Gattung Pterocarpus und der Gattung Baphia ausgewählt ist.

9. Zusammensetzung nach einem der vorhergehenden Ansprüche, **dadurch gekennzeichnet, dass** sie ein weiteres Farbmittel enthält, das unter den Nanopigmenten von Eisenoxid ausgewählt ist, deren mittlere Größe der Elementarpartikel unter 100 nm liegt.

10. Zusammensetzung nach einem der vorhergehenden Ansprüche, **dadurch gekennzeichnet, dass** sie mindestens einen kosmetischen Zusatzstoff enthält, der unter den Fettsubstanzen, organischen Lösungsmitteln, Emulgatoren, ionischen oder nichtionischen Verdickungsmitteln, beruhigenden Stoffen, Antioxidantien, Radikalfängern für freie Radikale, Trübungsmitteln, Stabilisatoren, Emollientien, Siliconen, α-Hydroxysäuren, Schaumverhütungsmitteln, Hydratisierungsmitteln, Vitaminen, Insekten abwehrenden Wirkstoffen, Substanz P-Antagonisten, entzündungshemmenden Wirkstoffen, Parfums, Konservierurigsmitteln, grenzflächenaktiven Stoffen, Füllstoffen, Polymeren, Treibmitteln, Alkalisierungsmitteln oder Ansäuerungsmitteln ausgewählt ist.

11. Zusammensetzung nach einem der vorhergehenden Ansprüche, **dadurch gekennzeichnet, dass** sie ferner mindestens ein organisches Lichtschutzmittel und/oder mindestens ein anorganisches Lichtschutzmittel, die im UV-A-und/oder UV-B-Bereich wirksam sind, enthält.

12. Zusammensetzung nach Anspruch 11, wobei das organische Lichtschutzmittel unter den 1,3,5-Triazinderivaten, Dibenzoylmethanderivaten, Zimtsäurederivaten, Anthranilaten, Salicylsäurederivaten, Campherderivaten, Benzophenonderivaten, β,β-Diphenylacrylatderivaten, Benzotriazolderivaten, Benzalmalonatderivaten, Benzimidazolderivaten, Imidazolinen, Bis-benzoazolylderivaten, p-Aminobenzoesäurederivaten (PABA), Methylen-bishydroxyphenylbenzotriazolderivaten, polymeren Filtern und Siliconfiltern, Dimeren, die von α-Alkylstyrol abgeleitet sind, und 4,4-Diarylbutadienen und deren Gemischen ausgewählt ist.

13. Zusammensetzung nach Anspruch 12, wobei das organische Lichtschutzmittel ausgewählt ist unter:
- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalydene Dicamphor Sulfonic Acid,
- Benzophenone-3,
- Benzophenone-4,
- Benzophenone-5,
- 4-Methylbenzylidene Camphor,
- Benzimidazilate,
- Anisotriazine,
- Ethylhexyl triazone,
- Diethylhexyl Butamido Triazone,
- Methylene bis-Benzotriazolyl Tetramethylbutylphenol,
- Drometrizole Trisiloxane,
- 2,4,6-Tris-(diisobutyl-4'-aminobenzalmalonat)-s-triazin
und deren Gemischen.

14. Zusammensetzung nach Anspruch 11, wobei das anorganische Lichtschutzmittel unter den Pigmenten oder Nanopigmenten von Metalloxiden, die umhüllt oder nicht umhüllt sind, ausgewählt ist.

15. Zusammensetzung nach Anspruch 14, wobei das anorganische Lichtschutzmittel unter den umhüllten oder nicht umhüllten Nanopigmenten von Titanoxid, Eisenoxid, Zinkoxid, Zirconiumoxid oder Ceroxid und deren Gemischen ausgewählt ist.

16. Zusammensetzung nach einem der Ansprüche 11 bis 15, wobei das Lichtschutzmittel in den Zusammensetzungen in Mengenanteilen von 0,1 bis 20 Gew.-%, bezogen auf das Gesamtgewicht der Zusammensetzung, und vorzugsweise im Bereich von 0,2 bis 15 Gew.-%, bezogen auf das Gesamtgewicht der Zusammensetzung, enthalten ist.

17. Zusammensetzung nach einem der vorhergehenden Ansprüche, **dadurch gekennzeichnet, dass** sie 0,5 bis 50 % und vorzugsweise 5 bis 30 % N-acyliertes Aminosäureescerderivat, bezogen auf das Gesamtgewicht der Zusammensetzung, enthält.

18. Zusammensetzung nach einem der vorhergehenden Ansprüche, **dadurch gekennzeichnet, dass** sie als nichtionische Vesikeldispersion, Emulsion und insbesondere Emulsion vom Wasser-in-Öl-Typ oder Öl-in-Wasser-Typ, als Creme oder als dreifache Emulsion (W/O/W oder O/W/O), Milch, Gel, Gelcreme, Suspension, Dispersion, Schaum oder Spray vorliegt.

19. Verwendung einer Zusammensetzung nach einem der Ansprüche 1 bis 18 als kosmetische Zusammensetzung, die für die Bräunung oder Braunfärbung der Haut vorgesehen ist.

20. Kosmetisches Verfahren zum Bräunen oder zur Braunfärbung der Haut, **dadurch gekennzeichnet, dass** es darin besteht, auf die Haut eine wirksame Menge einer kosmetischen Zusammensetzung nach einem der Ansprüche 1 bis 18 aufzutragen.

21. Verwendung mindestens eines N-acylierten Aminosäureesters der Formel
R'₁(CO)N(R'₂)CH(R'₃)(CH₂)ₙ(CO)OR'₄,
worin bedeuten:
n 0,1 oder 2,
R'₁ eine geradkettige oder verzweigte Alkyl- oder Alkenylgruppe mit 5 bis 21 Kohlenstoffatomen,
R'₂ ein Wasserstoffatom oder eine Alkylgruppe mit 1 bis 3 Kohlenstoffatomen, R'₃ eine Gruppe, die unter Wasserstoff, Methyl, Ethyl oder einer geradkettigen oder verzweigten C₃- oder C₄-Alkylgruppe ausgewählt ist,
R'₄ eine geradkettige oder verzweigte Alkylgruppe mit 1 bis 10 Kohlenstoffatomen oder eine geradkettige oder verzweigte Alkenylgruppe mit 2 bis 10 Kohlenstoffatomen oder einen Sterinrest,
in einer Zusammensetzung für die Bräunung und/oder künstliche Braunfärbung der Haut, die mindestens ein Selbstbräunungsmittel enthält, um die Färbung und/oder die Stabilität des Selbstbräunungsmittels zu verbessern.

## Claims

1. Cosmetic and/or dermatological composition, **characterized in that** it contains, in a cosmetically acceptable support:
(i) at least one self-tanning agent,
(ii) at least one ester chosen from N-acyl amino acid esters of formula
R'₁(CO)N(R'₂)CH(R'₃)(CH₂)ₙ(CO)OR'₄
in which:
n is an integer equal to 0, 1 or 2,
R'₁ represents a linear or branched C₅ to C₂₁ alkyl or alkenyl radical,
R'₂ represents a hydrogen atom or a C₁ to C₃ alkyl group,
R'₃ represents a radical chosen from the group formed by a hydrogen atom, a methyl group, an ethyl group and a linear or branched C₃ or C₄ alkyl chain,
R'₄ represents a linear or branched C₁ to C₁₀ alkyl radical or a linear or branched C₂ to C₁₀ alkenyl radical or a sterol residue.

2. Composition according to Claim 1, **characterized in that** the amino acid ester is isopropyl N-lauroyl sarcosinate
CH₃-(CH₂)₁₀CO-N(CH₃)-CH₂-COO-CH₂-(CH₃)₂.

3. Composition according to one of the preceding claims, such that the self-tanning agent is a mono- or polycarbonyl compound.

4. Composition according to Claim 3, such that the self-tanning agent is chosen from the group formed by isatin, alloxan, ninhydrin, glyceraldehyde, mesotartaric aldehyde, glutaraldehyde, erythrulose, pyrazoline-4,5-dione derivatives, dihydroxyacetone (DHA) and 4,4-dihydroxypyrazoline-5-dione derivatives.

5. Composition according to Claim 4, such that the self-tanning agent is DHA.

6. Composition according to any one of Claims 1 to 5, such that the concentration of self-tanning agents ranges from 0.1% to 10% by weight relative to the total weight of the composition.

7. Composition according to one of the preceding claims, **characterized in that** it comprises at least one synthetic or natural direct dye and/or at least one indole derivative.

8. Composition according to one of the preceding claims, such that it comprises an additional colouring agent chosen from "insoluble" extracts of red woods of the genus Pterocarpus and of the genus Baphia.

9. Composition according to one of the preceding claims, such that it comprises an additional colouring agent chosen from iron oxide nanopigments, the mean size of the elementary particles of which is less than 100 nm.

10. Composition according to any one of the preceding claims, **characterized in that** it comprises at least one cosmetic adjuvant chosen from fatty substances, organic solvents, emulsifiers ionic or nonionic thickeners, softeners, antioxidants, free-radical scavengers, opacifiers, stabilizers, emollients, silicones, α-hydroxy acids, antifoams, moisturizers, vitamins, insect repellants, substance P antagonists, antiinflammatories, fragrances, preserving agents, surfactants, fillers, polymers, propellants and acidifying or basifying agents.

11. Composition according to any one of the preceding claims, **characterized in that** it moreover also comprises at least one organic photoprotective agent and/or at least one inorganic photoprotective agent that is (are) active in the UVA and/or UVB range.

12. Composition according to Claim 11, such that the organic photoprotective agent is chosen from 1,3,5-triazine derivatives, dibenzoylmethane derivatives, cinnamic derivatives, anthranilates; salicylic derivatives, camphor derivatives; benzophenone derivatives; β,β-diphenylacrylate derivatives, benzotriazole derivatives; benzalmalonate derivatives; benzimidazole derivatives; imidazolines; bis-benzazolyl derivatives; p-aminobenzoic acid (PABA) derivatives; methylene bis(hydroxyphenyl)benzotriazole derivatives; screening polymers and screening silicones; dimers derived from α-alkylstyrene and 4,4-diarylbutadienes, and mixtures thereof.

13. Composition according to Claim 12, such that the organic photoprotective agent is chosen from:
- Ethylhexyl salicylate,
- Butyl methoxydibenzoylmethane,
- Ethylhexyl methoxycinnamate,
- Octocrylene,
- Phenylbenzimidazolesulphonic acid,
- Terephthalylidenedicamphorsulphonic acid,
- Benzophenone-3,
- Benzophenone-4,
- Benzophenone-5,
- 4-Methylbenzylidenecamphor,
- Benzimidazilate,
- Anisotriazine,
- Ethylhexyltriazone,
- Diethylhexylbutamidotriazone,
- Methylenebis(benzotriazolyl)tetramethylbutylphenol,
- Drometrizole trisiloxane,
- 2,4,6-tris(diisobutyl 4'-aminobenzalmalonate)-s-triazine,
and mixtures thereof.

14. Composition according to Claim 11, such that the organic photoprotective agent is chosen from coated or uncoated metal oxide pigments or nanopigments.

15. Composition according to Claim 14, such that the organic photoprotective agent is chosen from coated or uncoated nanopigments of titanium oxide, of iron oxide, of zinc oxide, of zirconium oxide and of cerium oxide, and mixtures thereof.

16. Composition according to any one of Claims 11 to 15, such that the organic photoprotective agent is present in the compositions in proportions ranging from 0.1% to 20% by weight relative to the total weight of the composition, and preferably ranging from 0.2% to 15% by weight relative to the total weight of the composition.

17. Composition according to one of the preceding claims, **characterized in that** it comprises from 0.5% to 50% and preferably from 5% to 30% of N-acyl amino acid ester derivative by weight relative to the total weight of the composition.

18. Composition according to one of the preceding claims, such that it is in the form of a nonionic vesicular dispersion, an emulsion, in particular an emulsion of water-in-oil type or of oil-in-water type, a cream or a triple emulsion (W/O/W or O/W/O emulsion), a milk, a gel, a cream-gel, a suspension, a dispersion, a mousse or a spray.

19. Use of a composition according to Claim 18 as a cosmetic composition for tanning or browning the skin.

20. Cosmetic process for tanning or browning the skin, **characterized in that** it consists in applying to the skin an effective amount of a cosmetic composition according to one of Claims 1 to 18.

21. Use of at least one N-acyl amino acid ester of formula
R'₁(CO)N(R'₂)CH(R'₃) (CH₂)ₙ(CO)OR'₄
in which:
n is an integer equal to 0, 1 or 2,
R'₁ represents a linear or branched C₅ to C₂₁ alkyl or alkenyl radical,
R'₂ represents a hydrogen atom or a C₁ to C₃ alkyl group,
R'₃ represents a radical chosen from the group formed by a hydrogen atom, a methyl group, an ethyl group and a linear or branched C₃ or C₄ alkyl chain,
R'₄ represents a linear or branched C₁ to C₁₀ alkyl radical or a linear or branched C₂ to C₁₀ alkenyl radical or a sterol residue,
in a composition for artificially tanning and/or browning the skin, containing at least one self-tanning agent, with the aim of improving the coloration and/or stability of the said self-tanning agent.
